# EUROPÄISCHE PATENTSCHRIFT

(11) **EP 0 767 239 B1**
(45) Veröffentlichungstag und Bekanntmachung des Hinweises auf die Patenterteilung: **04.12.2002**
(21) Anmeldenummer: 96115222.0
(22) Anmeldetag: 23.09.1996
(51) Int. Cl.: C12P 19/30, C07H 19/04, C12N 11/18

(54) **Verfahren zur enzymatischen Synthese von Nukleotid-6-desoxy-D-xylo-4-hexulosen**
Process for the enzymatic synthesis of nucleotide-6-desoxy-D-xylo-4-hexuloses.
Procédé de préparation enzymatique de nulcotide-6-desoxy-D-xylo-4-hexuloses.

(30) Priorität: 06.10.1995 DE 19537217
(43) Veröffentlichungstag der Anmeldung: 09.04.1997
(73) Patentinhaber: Aventis Research & Technologies GmbH & Co. KG, 65926 Frankfurt am Main (DE)
(72) Erfinder: Marquardt, Rüdiger, Dr., 60389 Frankfurt (DE); Hörsch, Brigitte, Dr., 65719 Hofheim (DE); Seiffert-Störiko, Andreas, Dr., 65929 Frankfurt (DE); Stein, Andreas, DCh., 52428 Jülich (DE); Zervosen, Astrid, DCh., 4840 Welkenraedt (BE); Elling, Lothar, Dr., 52066 Aachen (DE); Kula, Maria Regina, Prof. Dr., 52382 Niederzier-Hambach (DE); Verseck, Stefan, DB., 42103 Wuppertal (DE); Distler, Jürgen, DB., 42107 Wuppertal (DE); Piepersberg, Wolfgang, Prof. Dr., 42349 Wuppertal (DE)
(74) Vertreter: Ackermann, Joachim, Dr.

(56) Entgegenhaltungen:
- DE-C- 4 221 595
- ZERVOSEN A. ET AL.: "Combined enzymatic synthesis of nucleotide (deoxy) sugars from sucrose and nucleoside monophopshates" TETRAHEDRON, Bd. 52, Nr. 7, 1996, Seiten 2395-2404, XP002060321
- ZERVOSEN A. ET AL.: "Continuous enzymatic synthesis of 2'-deoxythymidine-5'-(alpha-D--glucopyrano syl)diphosphate" ANGEW. CHEM. INT. ED. ENG., Bd. 33, Nr. 5, 1994, WEINHEIM, DE, Seiten 571-572, XP002060326
- ELLING L. ET AL.: "Investigation of sucrose synthase from rice for the synthesis of various nucleotide sugars and saccharides" GLYCOBIOLOGY, Bd. 3, Nr. 4, August 1993, Seiten 349-355, XP002060322

## Beschreibung

Die vorliegende Erfindung betrifft ein Verfahren zur enzymatischen Synthese von Nukleotid-6-desoxy-D-xylo-4-hexulosen, ausgehend von einem Nukleosidmonophosphat (NMP).

Enzymatische Synthesen von Oligosacchariden, z. B. von N-Acetyllactosamin (LacNAc) und seinen Derivaten mittels Glycosyltransferasen, z. B. der β-1 4 Galactosyltransferase (GalT) [EC 2.4.1.38], und Nukleosidphosphatglycoside (Nukleotidzuckern) als Glycosyldonoren sind seit langem bekannt (Wong, C.-H., Ichikawa, Y., Krach, T., Gautheron-Le Narver, Ch., Dumas, D. P., Look, G. C., J. Am. Chem. Soc. 118, 8137 - 8145, 1991). Wong et al. (Wong, C.-H., Haynie, S. L., Whitesides, G. M., J. Org. Chem. 47, 5416 - 5418, 1982; Wong, C.-H., Wang, R., Ichikawa, Y., J. Org. Chem. 57, 4343 - 4344, 1992) entwickelten LacNAc- Synthesen bei welchen der Nukleotidzucker, die Uridindiphosphatglucose (UDP-Glc) in situ regeneriert wird. Infolgedessen ist es bei diesem Reaktionen nicht mehr notwendig, die relativ teuren Nukleotidzucker in stöchiometrischem Mengen einzusetzen.

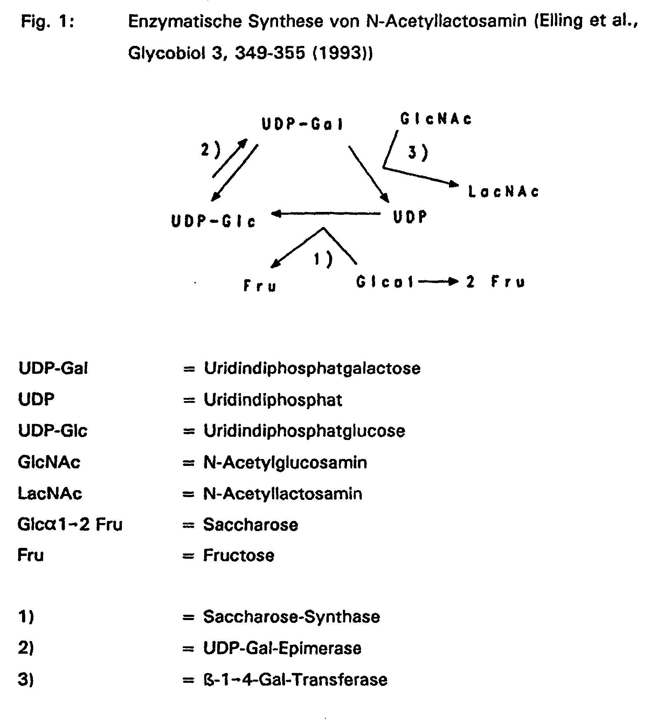

Der von Elling und Kula (DE P 42 21 595 C1; Elling, L., Grothus, M., Kula, M.-R., Glycobiol. 3, 349 - 355, (1993)) entwickelte LacNAc-Zyklus (Fig. 1) stellt gegenüber den bisher bekannten Zyklen insofern eine Verbesserung dar, daß nur drei anstelle von sechs Enzymen zur Synthese eingesetzt werden müssen. Die synthetisierten Disaccharide können als Edukte für weitere Umsetzungen mit unterschiedlichen Transferasen z. B. Sialyltransferasen oder Fucosyltransferasen verwendet werden.

Ein Zielprodukt dieser enzymatischen Synthesen ist beispielsweise das Tetrasaccharid Sialyl LewisX oder dessen Derivate (Ichikawa, Y., Lin, Y.-C., Dumas, D. P., Shen, G.-J.,Gracia-Junceda, E., Williams, M. A., Bayer, R., Ketcham, C., Walker, L. E., Paulson, J. C., Wong, C.-H., J. Am. Chem. Soc. 114, 9283 - 9298, 1992), dessen Bedeutung bei der Zell-Zell-Erkennung Gegenstand einer intensiven Forschung ist (DeFrees, S., Kosch, W., Way, W., Paulson, J. C., Sabesan, S., Halcomb, R. L., Huang, D.-H., Ichikawa, Y., Wong, C.-H., J. Am. Chem. Soc. 117, 66 - 79, 1995).

Die Saccharose-Synthase [EC 2.4.1.13] ist eine insbesondere in Pflanzen weit verbreitete Glycosyltransferase, deren Funktion als Katalysator zur Bildung von Nukleotidzuckern innerhalb des Stoffwechsels der Pflanze (Avigad, G., Encyclopedia of Plant Physiology New Series Vol. 13A, Carbohydrates I, Intracellular Carbohydrates, Springer Verlag, Berlin, 217-347, 1982) zusammenfassend dargestellt wurde. Das Enzym ist zur Synthese von Nukleotidzuckern, z. B. UDP-, dTDP-, ADP-, CDP- und GDP-Glc, geeignet (Elling, L., Grothus, M., Kula, M.-R., Glycobiol. 3, 349-355, 1993). Die Aufreinigung der Saccharose Synthase aus Reis (Elling, L., Kula, M.-R., J. Biotechnol. 29, 277 - 286, 1993), und ihr Einsatz zur in situ-Regeneration von UDP-Glc wurde von Elling et al. (DE P 42 21 595 C1; Elling, L., Grothus, M. and Kula, M.-R., Glycobiol. 3, 349-355, 1993) beschrieben. Das Reisenzym ist ein homotetrameres Protein mit einem Molekulargewicht von 362 kDa. Das Enzym wurde bereits zur präparativen Synthese von dTDP-Glc ausgehend von dTDP im Enzymmembranreaktor (EMR) eingesetzt (Zervosen, A., Elling, L., Kula, M.-R., Angew. Chem. 106, 592 - 593, 1994).

Aus Angew. Chem. Int. Ed. Eng., Bd. 33, Nr. 5, 1994, S. 571-2 (Zervosen A. et al., "Continuous enzymatic synthesis of 2'-deoxythymidine-5'-(alpha-D-glucopyranosyl)-diphosphate") ist ein Verfahren bekannt, bei dem ein Nukleosiddiphosphat (NDP) verwendet wird.

Die Kopplung von Reaktionen einer Kinase mit der Saccharose-Synthase ermöglicht eine wirtschaftlich günstigere Synthese von Nukleotidzuckern, da Nukleosidmonophosphate (NMP) gegenüber -Nukleosiddiphosphaten (NDP) deutlich preisgünstiger zu erhalten sind.

Präparative Synthesen von Nukleotidzuckern, ausgehend von einem Nukleosidmonophosphat (NMP) wurden bereits beschrieben (Heidlas J. E., Williams K. W., Whitesides G. M., Acc. Chem. Res., 25., 307 - 314, 1992). Hier setzte man das NMP zunächst unter Einsatz von Myokinase und Pyruvat-Kinase zum Nukleosidtriphosphat um, das dann mit dem jeweiligen Zucker-1-phosphat unter Katalyse einer spezifischen Pyrophosphorylase zum Nukleotidzucker reagierte.
Im Gegensatz zu den sehr spezifischen Pyrophosphorylasen kommt mit der Saccharose Synthase ein Enzym zum Einsatz, mit dem verschieden aktivierte Glucosedonoren hergestellt werden können.

Es wurde nun festgestellt, daß die Saccharose-Synthase neben den Nukleotiddiphosphaten (NDP) auch Desoxy-Nukleotiddiphosphate (dNDP) als Substrat akzeptieren kann. So kann z. B. dUDP-Glucose ausgehend von Saccharose als Glycosyl-Donor und UDP präparativ hergestellt werden.

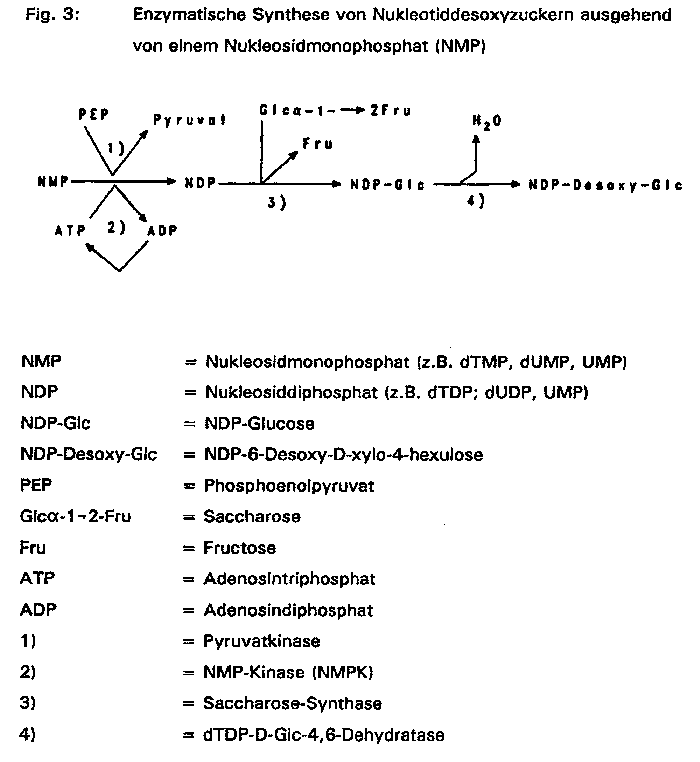

Es wurde ferner festgestellt, daß die Saccharose-Synthase mit anderen Enzymen gleichzeitig inkubiert werden kann und sich somit einfache und neuartige Herstellmöglichkeiten für aktivierte Zucker eröffnen. Diese Erkenntnis ist erst durch eine verbesserte und damit vergrößerte Verfügbarkeit der Saccharose- Synthase ermöglicht worden, wie sie in den Arbeiten von Elling beschrieben wurde (DE P 42 21 595 C1; Elling, L., Kula, M.-R., J. Biotechnol. 29, 277 - 286, 1993; Elling, L., Grothus, M. and Kula, M-R., Glycobiol. 3, 349-355, 1993). Das Enzym selbst ist seit mehreren Jahren bekannt, konnte jedoch mangels Menge bisher nicht auf proteinchemische Eigenschaften hin untersucht werden. So ist erst seit kurzem das pH-Optimum, sowie die für die Reaktion erforderlichen Ionen (lonenstärke, Anwesenheit von für die Reaktion unverzichtbaren Ionen) und auch die Regulation (Endprodukt-Hemmung) bekannt (L. Elling, Glycobiology 5, 201-206, 1995). Das Enzym ist zudem nicht kommerziell verfügbar, was eine Kombination mit den o. a. Enzymen nicht vereinfachte. Der Bedarf an Nukleotidzuckern als Substrate ist in den vergangenen Jahren durch die Entdeckung und Nutzung von Glycosyltransferasen gewachsen. Erst durch diese parallele Entwicklung entstand ein Bedarf, diese teuren Substrate möglichst kostengünstig herzustellen, um so leichter an definierte Glycosylstrukturen zu kommen. So können ausgehend von NMP oder dNMP teure NDP-Zucker bzw. dNDP-Zucker hergestellt werden. Ausgehend von NMP oder dNMP läßt sich, unter Zusatz einer Pyruvat-Kinase, NDP bzw. dNDP herstellen, welches von der Saccharose Synthase als Substrat erkannt und mit Glucose zu NDP-Glucose bzw. dNDP-Glucose verknüpft wird (Fig.3).

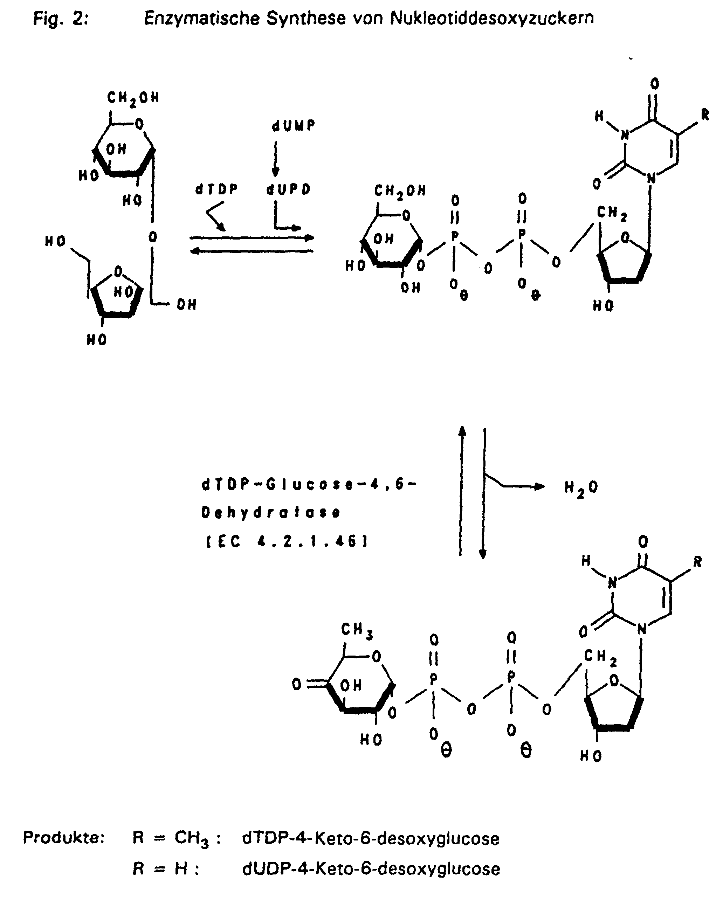

In Zellen werden einige primäre Nukleotidzucker enzymatisch in andere, sog. sekundäre Nukleotidzucker, umgewandelt. So entsteht aus GDP-Mannose beispielsweise GDP-Fucose in drei aufeinanderfolgenden Schritten. Einige neuere Erkenntnisse auf diesem Gebiet sind von Chang et al. am Beispiel der Schweine-Speicheldrüsen erarbeitet worden (J. Biol. Chem., 263, 1693-1697, 1988). Die Autoren beschreiben hier die Umwandlung von GDP-Mannose erst in GDP-4-Keto-6-Desoxy-Mannose mittels einer spezifischen Dehydratase und anschließend mittels einer Epimerase- und Reduktase-Aktivität zu GDP-Fucose. Die direkte Herstellung von Fucose ausgehend von Mannose kann im Stoffwechsel ohne Anwesenheit der Nukleotidgruppe nicht vollzogen werden. Diese Enzyme sind daher auf eine Aktivierung, d. h. den Nukleotidteil, angewiesen. Es ist vom derzeitigen Stand der Forschungen anzunehmen, daß die Bildung von anderen sekundären Nukleotidzuckern vergleichbar abläuft. Die Nukleotidzucker dUDP- und dTDP-6-Desoxy-D-Xylo-4-Hexulose werden unter Katalyse der dTDP-Glc-4,6-Dehydratase [EC 4.2.1.46] aus dUDP- und dTDP-Glc gebildet (Zarkowsky, H., Glaser, L., J. Biol. Chem. 222, 4750 - 4756, 1969). dTDP-6-Desoxy-D-Xylo-4-Hexulose ist ein Zwischenprodukt auf dem Biosyntheseweg der dTDP-L-Rhamnose. Die enzymatische Synthese und Isolierung dieser Substanz wurde beschrieben (Marumo, K., Lindqvist, L., Verma, N., Weintraub, A., Reeves, P. R., Lindberg, A. A., Eur. J. Biochem. 204, 539 - 545, 1992). dUDP-Glc ist nicht käuflich erhältlich, wurde jedoch mittels der dTDP-Glc-Pyrophosphorylase aus Pseudomonas aeruginosa in analytischen Mengen synthetisiert (Melo, A., Glaser, L., J. Biol. Chem. 240, 398 - 405, 1965) . Unter Nutzung des Synthesepotentials der Saccharose Synthase können dUDP- und dTDP-6-Desoxy-D-Xylo-4-Hexulose ausgehend von dUMP bzw. dTDP hergestellt werden (Fig. 2) (Stein, A., Dissertation, Heinrich Heine-Universität Düsseldorf, 1995).

Es wurde außerdem gefunden, daß die Saccharose Synthase zusammen mit weiteren Enzymen, die dUDP-Glc als Substrat benötigen, inkubiert werden kann. So kann z. B. eine NDP- bzw. eine dNDP-4,6-Dehydratase gleichzeitig mit Saccharose Synthase inkubiert werden, die deren Produkte weiter zu sekundären Nukleotidzuckern umsetzt. Die NDP- bzw. dNDP-4,6-Dehydratasen sind wichtige Enzyme für die Herstellung aktivierter Desoxyzucker, wie z. B. dTDP-L-Rhamnose.

Demnach betrifft die vorliegende Erfindung ein Verfahren zur enzymatischen Herstellung von Nukleosiddiphosphat-6-desoxy-D-xylo-4-hexulosen, welches sich dadurch auszeichnet, daß ein Nukleosidmonophosphat, Phosphoenolpyruvat, Adenosintriphosphat und Saccharose als Substrate sowie Pyruvatkinase, Nukleosidmonophosphatkinase, Saccharose-Synthase und Desoxythymidin-D-Glucose-4,6-Dehydratase als Enzyme in einer geeigneten Pufferlösung gleichzeitig inkubiert werden.

Als Nukleosidmonophosphat wird bevorzugt Desoxythymidinmonophosphat, Desoxyuridinmonophosphat oder Uridinmonophosphat eingesetzt. Gleichermaßen eignen sich die Monophosphate des Adenosins, des Cytidins sowie des Inosins.

Die Reaktion läuft in einer wäßrigen, gepufferten Lösung (beispielsweise Hepes, Tris, etc.) bei einem pH-Wert von 6-8, bevorzugt bei pH 7,2-7,5 ab. Die Temperatur beträgt 10 bis 40°C, bevorzugt 25°C, die Laufzeit beträgt 0-24h, bevorzugt 4h.

Das erfindungsgemäße Verfahren kann sowohl im Batch-Verfahren als auch kontinuierlich in einem Enzym-Membran-Reaktor (EMR) durchgeführt werden. Dabei ist der EMR vorzugsweise mit einer Ultrafiltrationsmembran mit einem Ausschlußvolumen kleiner als 30.000D ausgestattet, so daß die im Reaktionsgemisch enthaltenen Enzyme zurückgehalten werden, während die niedermolekularen Produkte (aktivierte Zucker) und nicht umgesetzte Edukte die Membran passieren und das Produkt im Auslauf detektiert werden kann. Vorzugsweise wird der Reaktor vor Gebrauch sterilisiert, so daß auf den Zusatz von antibakteriellen Substanzen (z. B. Azid), die möglicherweise auch die Enzymaktivität beeinträchtigen, verzichtet werden kann.

Das erfindungsgemäße Verfahren kann auch durch Perkolation der auf einen geeigneten pH-Wert eingestellten Lösung, enthaltend die Edukte dNMP oder NMP, bzw. dNDP oder NDP sowie Saccharose, über ein festes Trägermaterial, an das die entsprechenden Enzyme immobilisiert wurden, kontinuierlich durchgeführt werden. Als Trägermaterialien kommen dabei handelsübliche und dem Fachmann bekannte Träger (Eupergit, CNBr-aktivierte Sepharose, etc.) in Frage.

Ferner kann sowohl bei der Durchführung des Verfahrens in einem EMR, als auch bei der Perkolation der Lösung über die immobilisierten Enzyme die die Substrate enthaltende Pufferlösung zum wiederholten Kontakt mit den Enzymen in mehrfachen Zyklen zurückgeführt werden.

Die Aufarbeitung des Reaktionsgemisches und die Reinigung der erfindungsgemäß hergestellten dNDP- bzw. NDP-aktivierten Zucker erfolgt mit üblichen, aus dem Stand der Technik bekannten, Verfahren. Beispielsweise kann die Reaktionsmischung durch Filtration bzw. Zentrifugation geklärt und danach durch Ultrafiltration (Membran mit Ausschlußvolumen kleiner als 30.000D) das Enzym abgetrennt und restliches Produkt durch Diafiltration ausgewaschen werden. Die eigentliche Aufreinigung erfolgt dann z. B. mit gelchromatographischen Methoden, z. B. Gelchromatographie (z. B. Sephadex® oder Biogel), lonenaustausch- oder Dünnschichtchromatographie sowie HPLC oder dergleichen.

Die verfahrensgemäß verwendeten Ausgangsmaterialien, wie z. B. dNMP oder NMP sowie dNDP oder NDP und Saccharose und die verwendeten Enzyme sind im Handel erhältlich oder können nach in der Literatur beschriebenen Verfahren in an sich bekannter Weise hergestellt bzw. isoliert werden.

Die nach dem Verfahren gemäß der vorliegenden Erfindung hergestellten aktivierten Zucker können in an sich bekannter Weise als Bausteine für Glykokonjugate verwendet werden. Solche Glykokonjugate sind insbesondere im Pharma- und Agrobereich von Bedeutung. Hierbei dient der Nukleotid aktivierte Zucker als Substrat für eine geeignete Glykosyltransferase, welche den Zucker auf einen geeigneten Akzeptor überträgt. Die dNDP-aktivierten Zucker sind auch als Hemmstoffe für Glycosyltransferasen bekannt und können z. B. als Tumor- oder Virustatikum eingesetzt werden,

In folgenden wird das Verfahren gemäß der vorliegenden Erfindung anhand von Beispielen näher beschrieben.

Die HPLC-Einrichtung zur Analyse der Nukleotide und Nukleotidzucker besteht aus einer Hochpräzisionspumpe Modell 300 C, einem UV-Spektrophotometer SP-6V, einem automatischen Probensammler Modell CINA und einem Integrator Chromapac C-R GA (Gynkotek, München, BRD). Säulen: 2 x Aminex HPX-87H (300 x 7,8 mm) mit Kationen-H⁺-Austauscher Kartusche (30 x 4,6 mm); Eluens: 0,006M H₂SO₄. Fluß: 0,8 ml/min; Detektion: 205 nm.

### Beispiel 1: Präparative Synthese von dUDP-Glucose

### Syntheseansatz:

247mg dUMP (Na-Salz, 4mM, Sigma), 310mg PEP (Na-Salz, 6mM, Sigma), 27.3mg MgCl₂, 13,6mg ATP (Na-Salz, 0,12mM, Sigma), 25,7g Saccharose (500mM), Gesamtvolumen 150ml.
Puffer: Tris-HCI (100mM, pH7,2, 3mM DTT, 1mg/ml BSA)
Enzyme: 40U Saccharose-Synthase (2U/ml), 400U Pyruvatkinase (20U/ml), 20U NMPK (1U/ml).
Reaktor: 50ml Amicon®-Zelle mit YM30-Membran
Reaktionsvolumen: 20ml Lauf 1-6, 50ml Lauf 7
Inkubationstemperatur: 37°C

### Produktreinigung:

1) Spaltung von dUMP und dUDP mit der alkalischen Phosphatase [EC 3.1.3.1] aus Kälberdarm (Spezialqualität für die Molekularbiologie von Boehringer).
   - Ansatz:: 1 U alkalischen Phosphatase/ml Produktlösung
   17 Stunden bei 30°C.
2) Abtrennung der Proteine durch Ultrafiltration YM10
3) Ionenaustausch: Anionenaustausch an Q-Sepharose® FF (2,6 * 35,4 cm, v : 4 ml/min, Gradient: 0,5 l H₂O pH 7,0, 0,5 l 300 mM LiCl pH 7,0).
4) Einengen der Lösung am Rotationsverdamper bei 20-25 mbar und 35-40°C.
5) Entsalzung der Probe über G10 (2,6 * 93 cm, Elutionsmittel: Millipore® Wasser pH = 7,0)
6) Gefriertrocknung
7) Aufreinigung UDP-Glc

Für eine erneute Anionenaustauschchromatographie mit Q Sepharose® FF wurde der pH-Wert der Produktlösung auf 7,0 eingestellt. Durch den Einsatz eines linearen LiCl-Gradienten (0,25 l H₂O pH 7,0, 0,25 l 0,5 M LiCl pH 7,0) zur Elution des Produkts konnte das noch vorhandene Pyruvat abgetrennt werden (Enzymatischer Nachweis). Es folgten die Reinigungsschritte 4-6.

### Beispiel 2: Synthese von dUDP-6-Desoxy-D-Xylo-4-Hexulose ausgehend von dUDP-Glc

### Syntheseansatz:

| | |
|---|---|
| 20.1 mg | dUDPGIc (10mM) |
| 1920/µl | Hepes-NaOH (200mM, pH 7.2, 1mM DTT, 500mM Saccharose, 25mM KCI, 1mg/ml BSA) |
| 805µl | dTDP-D-Glc-4,6-Dehydratase (1.48U, Rohextrakt) |

Inkubation bei 30°C, Inkubationszeit: 4h

Nach 4h konnte kein dUDP-Glc mit der HPLC nachgewiesen werden. Die Synthese von dTDP-6-Desoxy-D-Xylo-4-Hexulose erfolgte unter analogen Versuchsbedingungen. Hier konnte eine vollständige Umsetzung bereits nach 1h beobachtet werden.

### Beispiel 3: Synthese von dUDP-6-Desoxy-D-Xylo-4-Hexulose ausgehend von dUMP

### Syntheseansatz:

V = 3 ml
4mM dUMP (Na-Salz, Sigma), 4mM PEP (CHA-Salz, Biomol), 0.8mM MgCl₂, 0,12mM ATP (Na-Salz, Sigma), 500mM Saccharose, 6U Saccharose-Synthase (2U/ml), 60 U Pyruvatkinase (20U/ml), 3U NMPK (1 U/ml), 15U dTDP-D-Glc 4,6-Dehydratase (5U/ml),
Puffer: Tris-HCl (100mM, pH 7.5, 3 mM DTT, 1mg/ml BSA, 50mM KCl)
Inkubationstemperatur: 25°C

Nach einer Inkubationszeit von 4h konnte die Bildung von 69.3 % des Produktes beobachtet werden.

## Patentansprüche

1. Verfahren zur enzymatischen Herstellung von Nukleotid-6-desoxy-D-xylo-4-hexulosen, **dadurch gekennzeichnet, daß** als Substrate ein Nukleosidmonophosphat, Phosphoenolpyruvat, Adenosintriphosphat und Saccharose sowie als Enzyme Pyruvatkinase, Nukleosidmonophosphatkinase, Saccharose-Synthase und Desoxythymidin-D-Glucose-4,6-Dehydratase in einer Pufferlösung gleichzeitig inkubiert werden.

2. Verfahren nach Anspruch 1, **dadurch gekennzeichnet, daß** als Nukleosidmonophosphat Desoxythymidinmonophosphat eingesetzt wird.

3. Verfahren nach Anspruch 1, **dadurch gekennzeichnet, daß** als Nukleosidmonophosphat Desoxyuridinmonophosphat eingesetzt wird.

4. Verfahren nach Anspruch 1, **dadurch gekennzeichnet, daß** als Nukleosidmonophosphat Uridinmonophosphat eingesetzt wird.

5. Verfahren nach einem der Ansprüche 1 bis 4, **dadurch gekennzeichnet, daß** das Verfahren als Batch-Verfahren durchgeführt wird.

6. Verfahren nach einem der Ansprüche 1 bis 4, **dadurch gekennzeichnet, daß** das Verfahren in einem Enzym-Membran-Reaktor kontinuierlich durchgeführt wird.

7. Verfahren nach einem der Ansprüche 1 bis 4, **dadurch gekennzeichnet, daß** die die Substrate enthaltende Pufferlösung über ein festes Trägermaterial kontinuierlich perkoliert wird, an welchem die Enzyme immobilisiert sind.

8. Verfahren nach Anspruch 6 oder 7, **dadurch gekennzeichnet, daß** die die Substrate enthaltende Pufferlösung zu einem wiederholten Kontakt mit den Enzymen mehrfach zyklisch zurückgeführt wird.

## Claims

1. A process for the enzymic preparation of nucleotide-6-deoxy-D-xylo-4-hexuloses, which comprises simultaneous incubation of a nucleoside monophosphate, phosphoenolpyruvate, adenosine triphosphate and sucrose as substrates, and pyruvate kinase, nucleosidemonophosphate kinase, sucrose synthase and deoxythymidine-D-glucose 4,6-dehydratase as enzymes in a buffer solution.

2. The process as claimed in claim 1, wherein deoxythymidine monophosphate is employed as nucleoside monophosphate.

3. The process as claimed in claim 1, wherein deoxyuridine monophosphate is employed as nucleoside monophosphate.

4. The process as claimed in claim 1, wherein uridine monophosphate is employed as nucleoside monophosphate.

5. The process as claimed in any of claims 1 to 4, wherein the process is carried out as a batch process.

6. The process as claimed in any of claims 1 to 4, wherein the process is carried out continuously in an enzyme membrane reactor.

7. The process as claimed in any of claims 1 to 4, wherein the buffer solution comprising the substrates is percolated continuously over a solid carrier material on which the enzymes are immobilized.

8. The process as claimed in claim 6 or 7, wherein the buffer solution comprising the substrates is recycled several times for repeated contact with the enzymes.

## Revendications

1. Procédé pour la préparation enzymatique de nucléotide-6-désoxy-D-xylo-4-hexuloses **caractérisé en ce que** l'on incube simultanément dans une solution tampon en tant que substrats un nucléoside monophosphate, un phosphoénolpyruvate, l'adénosine triphosphate et le saccharose ainsi que en tant qu'enzymes la pyruvate kinase, la nucléoside monophosphate kinase, la saccharose synthase et la désoxythymidine-D-glucose-4,6-déshydratase.

2. Procédé selon la revendication 1 **caractérisé en ce que** l'on emploie en tant que nucléoside monophosphate la désoxythymidine monophosphate.

3. Procédé selon la revendication 1 **caractérisé en ce que** l'on emploie en tant que nucléoside monophosphate la désoxyuridine monophosphate.

4. Procédé selon la revendication 1 **caractérisé en ce que** l'on emploie en tant que nucléoside monophosphate l'uridine monophosphate.

5. Procédé selon l'une des revendications 1 à 4 **caractérisé en ce que** l'on réalise le procédé comme un procédé en discontinu.

6. Procédé selon l'une des revendications 1 à 4 **caractérisé en ce que** l'on réalise le procédé en continu dans un réacteur enzymatique à membrane.

7. Procédé selon l'une des revendications 1 à 4 **caractérisé en ce que** l'on percole en continu la solution tampon contenant les substrats au travers un support solide sur lequel les enzymes sont immobilisées.

8. Procédé selon la revendication 6 ou 7 **caractérisé en ce que** l'on recycle en plusieurs cycles la solution tampon contenant les substrats en vue d'un contact répété avec les enzymes.
